(19) Europäisches Patentamt
European Patent Office
Office européen des brevets

(11) EP 4 542 560 A1

(12) EUROPEAN PATENT APPLICATION

(43) Date of publication:
23.04.2025 Bulletin 2025/17

(21) Application number: 24207459.9

(22) Date of filing: 18.10.2024

(51) International Patent Classification (IPC):
G16H 20/17 (2018.01)    A61B 5/145 (2006.01)
A61M 5/172 (2006.01)    G16H 40/63 (2018.01)

(52) Cooperative Patent Classification (CPC):
G16H 20/17; A61B 5/145; A61M 5/1723;
G16H 40/63

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 19.10.2023 US 202363591506 P

(71) Applicant: Insulet Corporation
Acton, MA 01720 (US)

(72) Inventor: NARAYANASWAMI, Rangarajan
Weston, MA, 02493 (US)

(74) Representative: Peterreins Schley
Patent- und Rechtsanwälte PartG mbB
Hermann-Sack-Straße 3
80331 München (DE)

(54) AUTOMATED MEDICAMENT DELIVERY SYSTEM ADJUSTMENTS WHEN TAKING A PHARMACEUTICAL

(57) Disclosed are methods, techniques, systems, and devices that are operable to compensate for the effects of pharmaceuticals on a user's analyte measurement values, such as glucose levels and/or ketone levels. The disclosed system is configured to enable medicament delivery application to enter a pharmaceutical mode. While in pharmaceutical mode, the medicament delivery application is operable to adjust medicament delivery constraints and other settings used by the medicament delivery application to respond to the effects of the pharmaceutical on the user's analyte measurement values.

FIG. 1A

EP 4 542 560 A1

**Description**

BACKGROUND

Different pharmaceuticals may cause physiological changes to a user. For a diabetic user, the physiological changes may be potentially harmful as some pharmaceuticals affect a user's glucose levels. Examples of pharmaceuticals that affect blood glucose levels include aspirin, antibiotics, and blood pressure medications such as ACE inhibitors, beta blockers, Norpace, Quinidine, steroids, such as corticosteroids, some forms of diuretics, supplements, such as niacin, chromium, vitamin E, St. John's wort, and the like, as well as other medicaments. For example, corticosteroids increase a patient's hepatic glucose production, lipolysis, and insulin resistance. In general, a person's blood glucose levels are affected (i.e., increased or decreased) by a regimen of pharmaceutical usage.

An automated medicament delivery (AMD) system may face problems when a diabetic patient is under a pharmaceutical treatment program and the AMD system is unable to deliver a higher insulin dose compared to baseline. This potentially results in hyperglycemia and carries risks of ketoacidosis because of constrained insulin delivery. In addition, adaptive AMD systems may also learn erroneous medicament requirements when the pharmaceuticals are taken on a temporary basis. For example, a diabetic user's insulin requirements may be altered by the pharmaceutical treatment program and, as a result, atypical blood glucose metrics and analyte sensor measurements may be produced.

Additionally, accommodating insulin dosing when a user (i.e., a person with diabetes) is under a pharmaceutical treatment program is a concern in the context of AMD systems.

BRIEF SUMMARY

This Summary is provided to introduce a selection of concepts in a simplified form that are further described below in the Detailed Description. This Summary is not intended to identify key features or essential features of the claimed subject matter, nor is it intended as an aid in determining the scope of the claimed subject matter.

In one aspect, a medicament delivery system, includes a processor. The medicament delivery system also includes a memory storing instructions including a medicament delivery application that, when executed by the processor, configures the processor to receive an indication to enter pharmaceutical mode, modify settings of the medicament delivery application in response to the received indication, where the modified settings are based on an anticipated increase in medicament amounts due to increased medicament resistance, and determine amounts of medicament to deliver to a user based on the modified settings.

In one aspect, a controller of a medicament delivery system, includes a processor. The controller also includes a memory storing instructions including a medicament delivery application that, when executed by the processor, configure the processor to receive a notification, while in a pharmaceutical mode of operation for the medicament delivery application, in response to the notification, calculate a bolus dosage using a first medicament delivery application setting and a second medicament delivery application setting, monitor analyte measurement signals with reference to one or more blood glucose metrics, based on the monitoring, modify the first medicament delivery application setting and modify the second medicament delivery application setting according to pharmaceutical mode settings, where the modified first medicament delivery application setting and the modified second medicament delivery application setting have an inverse relationship.

BRIEF DESCRIPTION OF THE DRAWINGS

To easily identify the discussion of any particular element or act, the most significant digit or digits in a reference number refer to the figure number in which that element is first introduced.

FIG. 1A illustrates an exemplary user interface for implementing one or more aspects of the disclosed subject matter.
FIG. 1B illustrates another exemplary user interface for implementing one or more aspects of the disclosed subject matter.
FIG. 1C illustrates yet another exemplary user interface for implementing one or more aspects of the disclosed subject matter.
FIG. 2 illustrates a flowchart of an exemplary process in accordance with the disclosed subject matter.
FIG. 3 illustrates an exemplary process flow for responding to a bolus request in accordance with the disclosed subject matter.
FIG. 4 illustrates a process flow for responding to a meal announcement while in pharmaceutical mode in accordance with the disclosed subject matter.
FIG. 5 illustrates an example process of when a user completes a pharmaceutical treatment program in accordance with the disclosed subject matter.

FIG. 6 illustrates a flowchart of an example of a learning process according to the disclosed subject matter.
FIG. 7 illustrates an exemplary medicament system operable to implement the examples disclosed herein.

DETAILED DESCRIPTION

Accommodating insulin dosing when the user is under some form of pharmaceutical therapy is a concern in the context of automated medicament delivery devices. It is known that while diabetic users are taking pharmaceuticals as well as certain over-the-counter medicines and supplements, hepatic glucose production increases while lipolysis (i.e., fat breakdown) and insulin resistance also increases, which leads to hyperglycemic conditions for type 1 and type 2 diabetics. Some pharmaceuticals may also decrease insulin resistance.

In some situations, insulin doses may need to increase approximately 30-70%. In some situations, insulin doses may need to be reduced. The following techniques, processes, devices, and systems are described with reference to a pharmaceutical mode implementation and medicament delivery under a pharmaceutical treatment program that may result in increases in hepatic glucose production, lipolysis, and insulin resistance; or in a decrease in hepatic glucose production, lipolysis, and insulin resistance. The pharmaceutical mode enables a user to explicitly set an operational mode of a medicament delivery application to a pharmaceutical mode which is operable to adjust AMD medicament delivery dosages. As an example, "pharmaceutical mode" may refer to an operating mode of a medicament delivery application that accounts for effects of a pharmaceutical on a user's physiology.

When the user has set the AMD system to pharmaceutical mode indicating that they are engaging in a pharmaceutical treatment program, the medicament delivery application (MDA) as described below may be operable to account for potential physiological changes and have the flexibility to delivery more insulin based on modified MDA settings as well as relaxed system constraints.

As mentioned earlier, different pharmaceuticals have different effects on a user's analyte levels, such as blood glucose levels, ketone levels and the like. As discussed herein, a pharmaceutical is a dietary supplement, medicament, medication, drug, or the like taken while the user (i.e., the diabetic user) is partaking in a pharmaceutical treatment program, and the one or more pharmaceuticals affects one or more of blood glucose levels, sensitivity to therapeutic medicaments (in particular insulin or GLP-1), ketone levels, and the like. The pharmaceuticals referred to herein, relate to substances other than those whose primary target is to reduce blood glucose levels, in particular insulin.

In an example in which a diabetic user (also referred to as "a user," or "a patient") in participating in a pharmaceutical treatment program in which the pharmaceutical is a corticosteroid, such as prednisone, studies have shown that increases in basal and bolus insulin doses are needed within six hours of the first dose of the pharmaceutical. However, in general, it has been found that a greater increase in insulin need may result during post prandial time periods. For example, a dose-dependent hyperglycemic effect appears to occur in patients within hours of exposure to the pharmaceutical because of the under-delivery of medicament, such as insulin, and the dose-dependent hyperglycemic effect is greater on postprandial blood glucose levels than fasting blood glucose levels. To compensate for the effect of the pharmaceutical, it has been estimated that insulin doses ought to be increased by at least 30% when the pharmaceutical treatment program is started and may need to be increased by approximately 70% or more to keep blood sugars in range. It would be beneficial if the increased insulin above the at least 30% increase was personalized for each user. When the pharmaceutical mode is set, insulin resistance is expected to increase and consequently the need for insulin increases. Conditions, other than increased insulin resistance, of the user may also be affected by a pharmaceutical treatment program and similar responses for delivery of medicament may be needed. Some of these conditions may also be addressed using the techniques, devices, and systems as described herein.

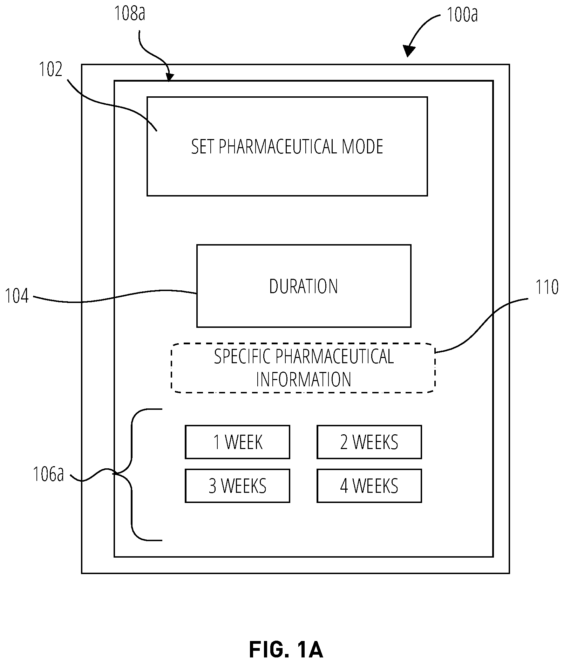

FIG. 1A illustrates examples of a user interface that enables a user to cause a medicament delivery application to enter a pharmaceutical mode of operation as described herein.

Patients with diabetes may set their mode to pharmaceutical mode when they are on this medication. The controller 100a includes a user interface 108a, a pharmaceutical mode button 102, a duration banner 104, and a duration setting 106a. For example, a user may interact with pharmaceutical mode button 102, which indicates to the MDA that the user has begun a pharmaceutical treatment program. The user has an option to interact with one of the duration settings 106a beneath the duration banner 104. The user interface 108a may be used to set the duration of the pharmaceutical mode in weeks that the user intends to use the pharmaceutical. The duration may also be set in hours, days or months.

In addition, the user interface 108a may optionally present a specific pharmaceutical information 110 selection button. The user can provide the specific pharmaceutical information, which may include at least one of: a name (e.g., the brand name, the clinical name, or the generic name) of the pharmaceutical, a type of the pharmaceutical, a dosage of the pharmaceutical, a frequency of dosages of the pharmaceutical, or quantity of pharmaceuticals, by selecting the specific pharmaceutical information 110. For example, another page of the user interface 108a may be presented for entering the specific pharmaceutical information. The name of the pharmaceutical may also be the chemical name, in particular the IUPAC name. The generic name may be the international nonproprietary name (INN), or country specific nonproprietary names, such as the United States Adopted Name, Dénomination Commune Française, or British Approved Name. In some embodiments, entering of the information may automated, for example, by the user scanning the medication packaging, or a machine readable code (i.e., QR-code) disposed thereon. The scanning of the packaging may be done with the controller 100a or a separate device in communication with the controller 100a, i.e., a smartphone.

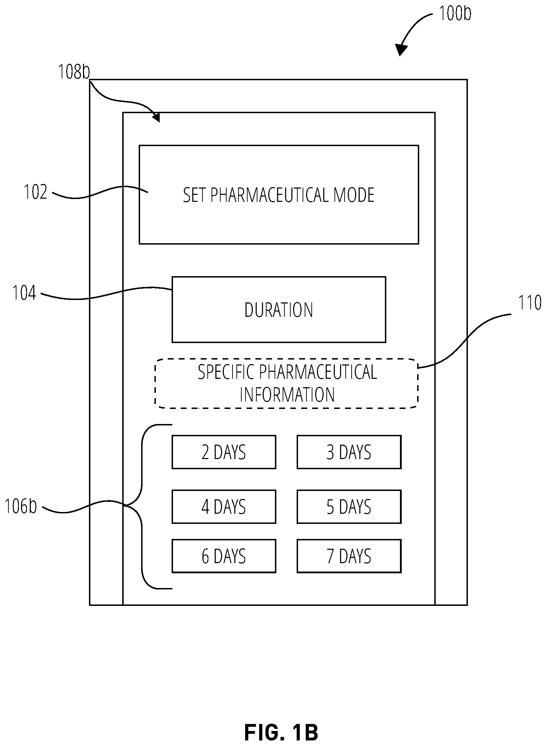

FIG. 1B illustrates another exemplary user interface for implementing one or more aspects of the disclosed subject matter.

The controller 100b includes user interface 108b, a pharmaceutical mode button 102, a duration banner 104, and a duration setting 106b. For example, a user may interact with pharmaceutical mode button 102, which indicates to the MDA that the user has begun a pharmaceutical treatment program. The user has an option to interact with one of the duration settings 106b beneath the duration banner 104. The user interface 108b may be used to set the duration of the pharmaceutical mode in days that the user intends to use the pharmaceutical.

In addition, the user interface 108b may optionally present a specific pharmaceutical information 110 selection button. The user can provide particular pharmaceutical information, such as pharmaceutical name, type of pharmaceutical, pharmaceutical dosage and frequency of administration to the MDA by selecting the specific pharmaceutical information 110. For example, another page of the user interface 108b may be presented for entering the particular pharmaceutical information.

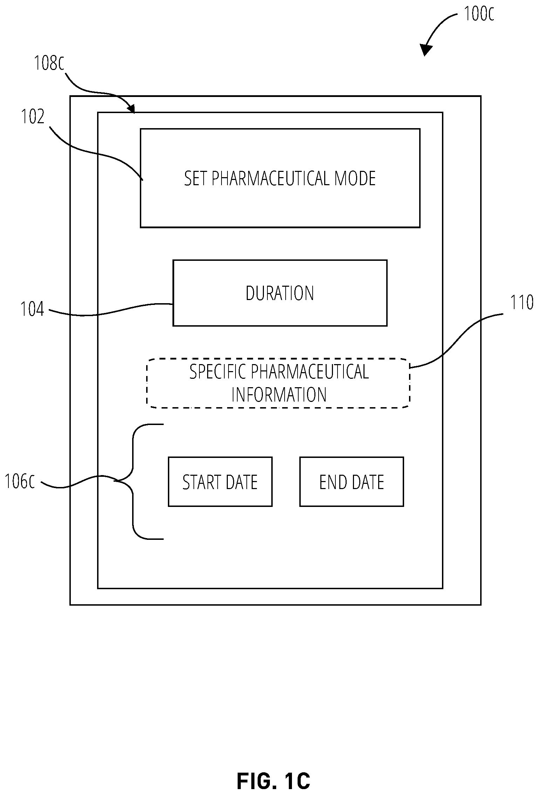

FIG. 1C illustrates yet another exemplary user interface for implementing one or more aspects of the disclosed subject matter. The controller 100c comprises a pharmaceutical mode button 102, a duration banner 104, and a duration settings 106c. The user interface 108c may be used to set the duration for date range extending from a "start date" to an "end date" that the user intends to use the pharmaceutical. Alternatively, the duration settings 106c may be a "start date" and the number of days the pharmaceutical is prescribed to be taken. For example, a user may interact with pharmaceutical mode button 102, which indicates to the MDA that the user has begun a pharmaceutical treatment program. The user has an option to interact with one of the duration settings 106b beneath the duration banner 104.

In addition, the user interface 108c may optionally present a specific pharmaceutical information 110 selection button. The user can provide particular pharmaceutical information, such as pharmaceutical name, type of pharmaceutical, pharmaceutical dosage and frequency of administration to the MDA by selecting the specific pharmaceutical information 110. For example, another page of the user interface 108c may be presented for entering the particular pharmaceutical information.

When the duration setting ends and the pharmaceutical mode is turned Off, the user interface may present reminders or alert(s) to the user that the pharmaceutical mode is shutting off as scheduled.

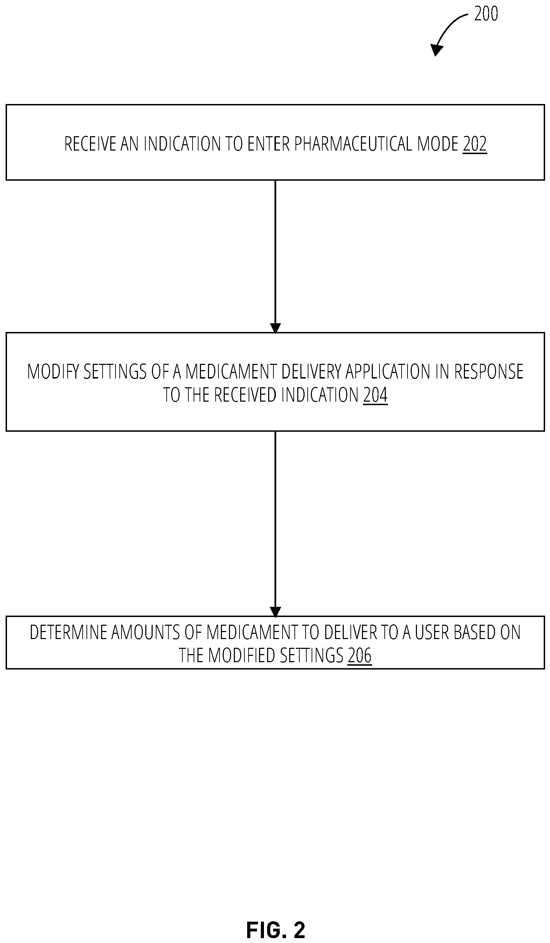

FIG. 2 illustrates a flowchart of an exemplary process in accordance with the disclosed subject matter. In block 202, a medicament delivery application (MDA) executed by a processor and that implements process 200 may be operable to receive an indication to enter pharmaceutical mode. For example, the user may select a pharmaceutical mode button 102 as described with reference to FIGs. 1a-1c above. The presence of a pharmaceutical in the blood of a user typically causes an increased medicament resistance. In an example, the indication may also inform the process 200 how long the user expects to remain in pharmaceutical mode by including a time duration of the pharmaceutical mode. The pharmaceutical mode time duration may be identified as lasting a selected number of days, weeks, months, or with a specific start date and a specific (or estimated) end date. The indication may also present the type of pharmaceutical, pharmaceutical dosage, and other specific pharmaceutical information. The MDA may be configured to react appropriately based on the pharmaceutical and/or the pharmaceutical mode time duration.

In block 204, the processor, in response to the received indication, modifies settings of the medicament delivery application in response. In an example, the modified settings may be based on an anticipated increase in medicament amounts due to increased medicament resistance caused by the pharmaceutical. In an example, the increased insulin need is compensated for by increasing the user's typical TDI setting.

As background, a traditional bolus calculator as well as meal announcement type request may address the increased insulin need.

In an example, a traditional bolus calculator may calculate bolus insulin needed as:

$$Correction = \left( \frac{Current\ BG - (Setpoint)}{CF} \right) (Equation\ 1),$$

where *CF* is a correction factor that may be given by the 1800 rule as 1800 divided by TDI (i.e., 1800/TDI). Other types of rules, such as the 1500, 1600 rules may also be used when determining the correction factor *CF*. One way to effectively increase insulin delivery is by decreasing the value of the correction factor either by choosing a value lower than 1800 or by increasing the TDI. The correction calculated in Equation 1 may be used to augment a meal dosage as follows:

$$meal\ dose = \frac{grams\ of\ carbs}{Insulin\ to\ Carbs\ Ratio} + Correction - Current\ Total\ IOB$$

(Equation 2).

where the current total IOB is the determined amount of insulin-on-board for the user.

In response to the pharmaceutical mode settings, the typical TDI value may be modified to provide a modified TDI. For example, the modified TDI may be a user's typical TDI increased by a set percentage, such as 5%, 10%, 12% or the like, at the initiation of the pharmaceutical mode. The modified TDI may be further increased as needed. So, in Equation 1, when the correction factor *CF* is determined, the modified TDI is used, which would be the user's typical TDI plus the set percentage increase. For example, the modified TDI in pharmaceutical mode may be approximately 110% of the user's typical TDI as compared to approximately 100% of the user's typical TDI when not in pharmaceutical mode.

Additionally, or alternatively, a user's typical insulin to carbohydrate ratio (ICR) may be reduced to provide a modified ICR. The modified ICR increases the meal dose in Equation 2 above, which is an increase in the amount of insulin delivered by bolus (i.e., the meal dose). In an example, the ICR may be a reduced by a set percentage, such as 5%, 10%, 12% or the like, at the initiation of the pharmaceutical mode.

Thus, at block 204, the MDA begins operating using the modified settings, such as the modified TDI and/or a modified ICR. Of course, other settings of the MDA, such as maximum insulin delivery (per day), insulin-on-board (IOB), or the like, may, additionally, or alternatively, be modified.

In block 206, the processor when executing the process 200 may be operable to determine amounts of medicament to deliver to a user based on the modified settings.

Additional actions that may be taken by the MDA based on the pharmaceutical mode are described with reference to the following examples.

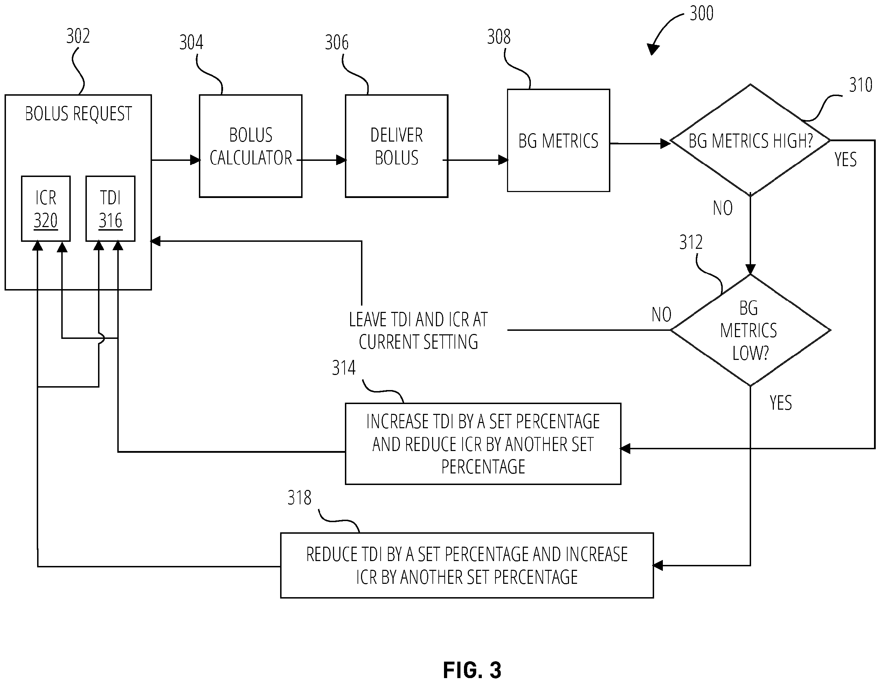

FIG. 3 illustrates a process flow of an exemplary process in accordance with the disclosed subject matter. As generally discussed with reference to FIG. 2, when the user has set pharmaceutical mode, the AMD system will take this into account and have the flexibility to deliver more insulin based on increasing the allowed Total Daily Insulin (TDI) or reducing the allowed insulin-to-carbohydrate ratio (ICR), as well as relaxing system constraints.

Based on the user's indication of pharmaceutical mode, the medicament delivery application (MDA) may modify one or more settings, one of those settings may be the TDI and another may be the ICR. The following discussion is based on the premise that the MDA is operating in a pharmaceutical mode. In response to the received indication that the user wishes to engage pharmaceutical mode, the MDA modifies the values of TDI and/or ICR by adding and subtracting a preset percentage to the current values of TDI and ICR, respectively. Of course, an alternative implementation is that the MDA makes modifications to the TDI and ICR based on the evaluation of glucose measurement values received after switching operation to the pharmaceutical mode.

According to some examples, the process 300 includes a number of steps and actions, such as receiving a notification from a user device (such as a controller), an input device, a third-party application, a cloud-based service, or the like. An example of a notification may be a bolus request 302. A request for a bolus is commonly received after ingestion of a meal, or post prandially, and by some users just prior to the meal. The bolus request 302 may be received via any one of a number of ways, such as a request for a bolus, or via a meal announcement details of which are described in a later example.

At the onset of the pharmaceutical mode (e.g., after receipt of the indication to enter pharmaceutical mode), the processor executing the MDA may modify the user's typical MDA setting for some constraints to provide modified MDA settings. Examples of MDA settings may be TDI 316 and ICR 320. For example, the values of TDI 316 and ICR 320 may be modified at the onset of pharmaceutical mode.

In response to receipt of the bolus request 302 of process 300, the MDA may utilize the modified settings of the medicament delivery application to determine a bolus dosage in response to receiving the bolus request 302. For example, the processor, in response to the bolus request 302, may retrieve modified values of TDI 316 and insulin-to-carbohydrate ratio (ICR) 320 from a memory coupled to the processor. The MDA executed by the processor may calculate a bolus dosage using, for example, a first medicament delivery application setting and a second medicament delivery application setting. For example, the modified values of TDI 316 and ICR 320 may be input into the bolus calculator 304 as the first medicament delivery application setting and the second medicament delivery application setting, respectively. The bolus calculator 304 may be a process executed by the processor as part of executing the MDA, or as a separate application that the MDA calls, such as a cloud-based service or another application maintained on a control device.

After the bolus dosage is calculated by the bolus calculator 304, the bolus dosage may be delivered at deliver bolus 306. The MDA continuously monitors the user's glucose level. After the bolus dosage is delivered at 306, the MDA monitors the user's glucose level in response to the delivered bolus dosage. The processor may begin, or continue, receiving analyte values from analyte sensors coupled to the user's body. Examples of analyte levels (also referred to as "analyte values") include glucose levels, ketone levels, glucose level trend indication, ketone level trend indication, and the like as well as other information, such as timestamp for the respective measurements, and the like.

The monitoring of the user's glucose level may be performed at BG metrics 308. When evaluating the received analyte levels with respect to the number of analyte metrics, the evaluating includes determining one or more of: a number of the received plurality of analyte values that are below a first threshold, a number of the received plurality of analyte values that are over a second threshold, a number of the received plurality of analyte values that are over a third threshold, a mean value of the received plurality of analyte in a first window of time, or a mean value of the received plurality of analyte in a second window of time.

For example, the MDA may flag the user's glucose level when the bolus dosage was delivered as the starting glucose level. The MDA may continue to monitor the user's glucose level with reference to glucose level metrics collected at BG metrics 308. The processor may receive analyte measurement signals from the analyte sensor(s), for example, every 1-5 minutes for 3-6 hours, 12-24 hours, or the like. For example, after a period of time, such as 6 hours, there may potentially be 72 analyte values that the processor may have evaluated or have to evaluate, and may be continuing to evaluate as more current analyte measurement signals are received.

An "analyte measurement signal" may refer to a raw data signal, a glucose measurement value, ketone measurement value, and/or other data or information output by an analyte sensor, such as a ketone sensor or a continuous glucose monitor (CGM). The raw data signal may be a measurement of an analyte by the sensor that needs to be interpreted by a receiving device. A glucose measurement value and ketone measurement value may be signals that have been interpreted by the analyte sensor and may have a particular format. The other data (uninterpreted) or information (interpreted) may be related to trends of the measurement values made by analyte sensor, time stamps, previous values, or the like.

In order to collect the BG metrics 308, the MDA may evaluate a user's glucose measurement values in 15 minute increments. For example, approximately 15 minutes after the starting glucose level is flagged and every approximately 15 minutes thereafter, the MDA may note the user's glucose measurement value based on data received from the analyte sensor. An analyte sensor may be operable to send an analyte measurement signal. "Analyte measurement signal" refers to a raw data signal, a glucose measurement value, ketone measurement value, and/or other data or information output by an analyte sensor, such as a ketone sensor or a continuous glucose monitor (CGM). The raw data signal may be a measurement of an analyte by the sensor that needs to be interpreted by a receiving device. A glucose measurement value and ketone measurement value may be signals that have been interpreted by the analyte sensor and may have a particular format. The other data (uninterpreted) or information (interpreted) may be related to trends of the measurement values made by analyte sensor, time stamps, previous values, or the like.

The raw data signal may have to be interpreted by the MDA as may information (e.g., timestamp, previous raw data signal value, or the like) included in the other data. The MDA may further evaluate the user's glucose measurement values by also determining the mean value of the user's glucose level every 15 minutes and every 30 minutes. Of course, time intervals other than 15 minutes and 30 minutes may be set, such as, for example, 10 minutes and 20 minutes, 5 minutes, and 15 minutes, or the like.

As part of the evaluating of the BG metrics 308, the MDA also may compare the user's glucose level obtained every 15 minutes to one or more thresholds and maintain a count of the number of times the user's glucose level either exceeds or falls below the one or more thresholds. For example, a continuous glucose monitor (CGM) may output an analyte measurement signal periodically, such as every 1 minute, every 5 minutes, every 10 minutes, or the like. The period for evaluating the received analyte measurement signals may be modified depending upon the user's activity or the time of day, for example, the period may be extended (e.g, from every 15 minutes to every 30 minutes, or longer) during a user's sleeping hours.

When evaluating the analyte measurement signals, the MDA may maintain a count of when the mean value of the user's glucose level received from a continuous glucose monitor (CGM) falls below a hypoglycemic threshold (e.g, less than 70 mg/dL), is greater than a first hyperglycemic threshold (e.g., greater than 180 mg/dL), or when the user's glucose level remains over a second hyperglycemic threshold (e.g., greater than 250 mg/dL). The respective hypoglycemic threshold, first hyperglycemic threshold, and second hyperglycemic thresholds may be set at default values, such as those listed above, and as time progresses may be customized to the user. For example, the user may indicate when they are feeling low (e.g., hypoglycemic) or high (e.g., hyperglycemic).

After a period of time, such as 15 minutes, 30 minutes, or for as long as 3 hours after the bolus request 302 (e.g., a typical length of a post-prandial period), or any other period of time between 15 minutes and 3 hours, the MDA may evaluate the BG metrics collected at BG metrics 308. The BG metrics that are gathered may be gathered over a period of time that, for example, may span 15 minutes, 3 hours, or a time therebetween, such as 30 or 60 minutes.

The processor may further evaluate the received analyte values with reference to different analyte metrics. For example, the received analyte values may be glucose levels, which are provided by a continuous glucose monitor (CGM), or the like. Based on results of the evaluation, the processor may be operable to adjust total daily medicament settings, such as total daily insulin (TDI), and medicament to carbohydrate ratio, such as insulin-to-carbohydrate ratio ICR.

The evaluating of the received plurality of analyte levels may be executed with respect to a plurality of analyte metrics. The analyte metrics may be collected by determining one or more of: a number of the received plurality of analyte values that are below a first threshold, a number of the received plurality of analyte values that are over a second threshold, a number of the received plurality of analyte values that are over a third threshold, or the like.

Additionally, a mean value of the received plurality of analyte values in a first window of time, or a mean value of the received plurality of analyte in a second window of time. A mean value of the analyte values is obtained during the first window or second window of time to lessen, or smooth, the effect of drastic or sudden changes in the collected analyte values. In an example, a first window of time may be 15 minutes, while a second window of time may be 30 minutes. These mean values may be compared to different glucose measurement thresholds, such as 70 mg/dL, 80 mg/dL, 90 mg/dL, 160 mg/dL, 180 mg/dL, 210 mg/dL, 250 mg/dL, or the like. The different glucose measurement thresholds may be the first threshold value, the second threshold value, and the third threshold value, respectively, as mentioned above. The values of the glucose measurement thresholds are selected so the processor may determine whether the user's blood glucose levels are trending toward hypoglycemia or hyperglycemia.

The processor may be operable to count the number of times the glucose measurement value does not meet a respective threshold (e.g., in the case of the lower glucose measurement values, such as a first threshold of approximately 70-90 mg/dL) or exceeds another respective threshold (e.g., in the case of the higher glucose measurement values, such as the approximately 160-210 mg/dL). Additionally, because the user's partaking in the pharmaceutical treatment program tends to cause the user's insulin need to increase, a third threshold, such as 250 mg/dL, may be set for evaluation of the user's glucose measurement values. In other words, the first threshold may be a hypoglycemia threshold, the second threshold may be a low hyperglycemia threshold and the third threshold may be a high hyperglycemia threshold.

In a detailed example, the processor may receive over the course of 1 hour a number of glucose measurement values of the user. The 1-hour may be divided into four 15-minute segments. The mean glucose level for each of the respective 15-minute segments may be compared to the different measurement value thresholds. If, for example, a processor determines a mean value of a first of the 4 segments is greater than 70 mg/dL (a first threshold), the processor may either count a zero value or no value for the first threshold. The processor may subsequently compare the mean value of the first of the four segments to the second threshold, which may be 180 mg/dL, if the mean value is greater than the second threshold, the processor may generate a count of 1 for greater than 180 mg/dL. The processor may subsequently compare the mean value of the first of the four segments to the third threshold, which may be 250 mg/dL. If the mean value is greater than the third threshold, the processor may generate a count of 1 for greater than 250 mg/dL. The processor may perform a similar process for segments 2-4, and produce a final threshold count for the 1-hour of time.

After collecting and evaluating the received analyte values with reference to the BG metrics for a period of time at 308, the processor may monitor analyte measurement signals with reference to one or more blood glucose metrics.

For example, the processor may determine, at 310, whether any of the BG metrics are low or high based on the total count of the number of time segments the respective first, the respective second threshold, and/or the respective third threshold was violated. For example, the post prandial BG metrics may be considered low when a mean of a set of BG glucose measurement values is less than a first threshold, such as < 70 mg/dL, in any segment. A BG metric may be considered high when the number of post prandial BG (i.e., glucose measurement value) readings is greater than a second threshold, such as > 180 mg/dL, is more than a preset number (for example, 6), and/or when the number of post prandial BG readings is greater than a third threshold, such as > 250 mg/dL, is more than another preset number (for example, 2), for example. The MDA may maintain a count of when each glucose measurement value or mean of several glucose measurement values violated the inequality related to the respective different thresholds.

In addition, the evaluation of the BG may also be evaluated against a user's baseline values from when the user is not taking pharmaceuticals (i.e., typical post prandial glucose levels when the MDA is not in pharmaceutical mode).

At 310, the MDA may identify, or may have identified, a number of times when the second and third glucose measurement thresholds are violated and compare the count of the number of times to respective count reference values, e.g., 6, 2, 1, 8, or the like. For example, the MDA may compare the count of the number of violations of the second and third glucose measurement thresholds to the respective count reference values for the second and third glucose measurement thresholds.

If the determination at 310 is "Yes, the BG metrics are high," the process 300 may proceed to increase TDI by a set percentage and reduce ICR by another set percentage 314. For example, the TDI 316, which had been increased by a set percentage at the initiation of the pharmaceutical mode to provide a modified TDI, may be further increased by the additional TDI increase percentage. The additional TDI increase percentage may be a value, such as 5%, 10%, 15% or 20%, that is selected depending upon the respective total counts of the number of segments (e.g., 15-minute segment or 30-minute segment) that exceeded the second threshold (e.g., > 180 mg/dL) and third threshold (e.g., > 250 mg/dL), respectively. For example, if the total count of the number of segments that exceeded the third threshold was equal to the total count of the number of segments that exceeded the second threshold, the user may potentially be hyperglycemic, in which case, the MDA may use a higher additional TDI increase percentage, such as 10% instead of 5%. Conversely, if the total count of the number of segments that exceeded the second threshold was greater than the total count of the number of segments that exceeded the third threshold, the additional TDI increase percentage may be 5 % or another value less than 10% because the user may not yet be hyperglycemic and may be heading toward being euglycemic. Once the modified TDI is adjusted by the additional TDI increase, the value may be saved as the TDI 316 in a memory by the MDA and may be used by the MDA as an input to the bolus calculator 304 in response to the next bolus request 302. The ICR 320 may be reduced by a preset percentage, which may be adjusted in a manner similar to that explained with reference to the adjustment of the percentage of TDI 316.

Other triggers for a "high BG metric" may include whenever any segment exceeds the third threshold (e.g., > 250 mg/dL) for a single count, whenever the count is greater than 2 for when the second threshold (e.g., > 180 mg/dL) is exceeded, or the like.

Alternatively, if, at 310, the determination is, "No, the BG metrics are not high," the process 300 proceeds to 312. At 312, the processor executing the MDA determines whether the BG metrics are low.

If the determination at 312 is "No, the BG metrics are not low either," the process 300 may proceed back to 302 where the values of TDI 316 and ICR 320 are left at their current setting, and the MDA waits for another bolus request.

Conversely, when the determination at 312 is "Yes, the BG metrics are low," the process 300 proceeds to 318. When the BG metrics are low, the MDA begins reducing the amount of insulin that is delivered in order to prevent further lowering the user's glucose level. At 318, the processor executing the MDA is operable to utilize pharmaceutical mode settings indicate that TDI is to be reduced by a set percentage and that ICR is to be increased by another set percentage. For example, the TDI 316 and/or ICR 320 may be adjusted by an additional TDI reduction percentage and an additional ICR increase percentage, respectively. The additional ICR increase percentage may be a value, such as 5%, 10%, 15% or 20%, depending upon the respective total count of the number of segments, while the TDI reduction percentage may be 10%, 15%, 20%, or the like.

In an example, at 318, the TDI 316 may be reduced and the ICR 320 may be increased by a larger percentage when the total number of counts is greater than some threshold, such as 3 out of 4 15-minute segments, some other ratio, or the like. While the discussion was based on the number of 15-minute segments of data from the analyte sensor, the individual data signals or individual glucose measurement values may also be used instead of the mean of the glucose measurement values over a 15-minute segment.

In summary of the 314 and 318, the processor, based on the monitoring of BG metrics at 310 and 312, may modify the first medicament delivery application setting and modify the second medicament delivery application setting, wherein the modified first medicament delivery application setting and the modified second medicament delivery application setting have an inverse relationship.

The MDA may also be operable to maintain a count based on a mean value of 30-minute segments. A purpose for taking the mean of the glucose measurement values over a time segment, whether 15, 20 or 30 minutes, is to provide some stability to the glucose measurement values and compensate for any errors or missing values or the like. In addition, the longer time segments, such as the 30 minute segments, may be selected when the indicated duration of the pharmaceutical treatment program is indicated via the duration settings 106a, 106b or 106c is indicated to be greater than 2 days, for example, or the duration is indicated as 1 week, or longer. In addition, while the above discussion of the BG metric evaluation was described with reference to 15-minute and 30-minute segments, the BG metric evaluation may also be conducted using each individual glucose measurement value. In such a case, the respective count thresholds may be adjusted. For example, instead of two counts greater than 250 mg/dL generating a response, the number of counts when individual glucose measurement values are being evaluated may be increased to 3, 4, 6, or the like.

The process 300 may be executed whenever a bolus is requested. Alternatively, the process 300 may be executed for the first bolus of the day or for the last bolus of the day, or whenever a second bolus is requested, or some other time related to a bolus request.

Although the example process 300 depicts a particular sequence of operations, the sequence may be altered without departing from the scope of the present disclosure. For example, some of the operations depicted may be performed in parallel or in a different sequence that does not materially affect the function of the process 300. In other examples, different components of an example device or system that implements the process 300 may perform functions at substantially the same time or in a specific sequence.

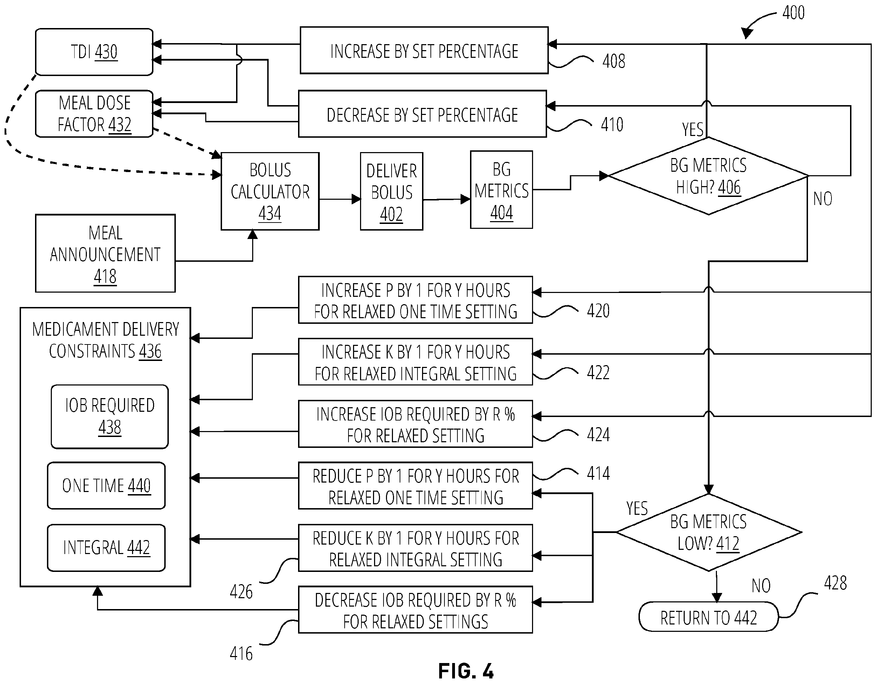

FIG. 4 illustrates a process flow for responding to a meal announcement while in pharmaceutical mode in accordance with the disclosed subject matter.

The process 400 may be implemented after initiation of the pharmaceutical mode by a user. A purpose of FIG. 4 is to illustrate a meal announcement process 400 while in pharmaceutical mode.

According to some examples, the process 400 includes a number of steps and actions, such as receiving a notification from a user device (such as a controller), an input device, a third-party application, a cloud-based service, or the like. An example of a notification may be a meal announcement. Initiation of the meal announcement process 400 while in pharmaceutical mode begins when the MDA receives a meal announcement 418. The meal announcement 418 may be received either from a user via a user interface, from an automated meal identification application, or the like. The meal announcement 418 may provide additional information, such as an estimated number of grams/ounces of carbohydrates, a meal or drink identification, or the like. An "automated meal identification application" refers to an application that is operable to determine whether a diabetic user has ingested food by one or more processes, such as through a change in blood glucose measurement values that has been identified to occur when the user eats, a calendar setting (e.g., a dinner date appointment, picnic appointment, happy hour appointment, or the like), an image recognition application (e.g., a food detection application, a restaurant recognition application, or the like), a location recognition application (e.g., a global positioning system mapping application), a combination thereof, or the like.

Upon receipt of the meal announcement 418, the MDA may call a bolus calculator 434 to calculate a bolus dosage to address the ingested meal. The bolus calculator 434 may be an MDA routine hosted on a user's controller (e.g., smartphone) or a third-party application also hosted on the user's controller or via a cloud-based service. Alternatively, the bolus calculator 434 may be hosted by a processor of a wearable medicament delivery device or be distributed between a controller and the wearable medicament delivery device, or the like. The meal announcement 418 may, for example, provide an estimated number of grams of carbohydrates to the bolus calculator 434.

The bolus calculator 434 may be operable to calculate a bolus dosage using not only the estimated number of grams of carbohydrates, but also the TDI 430 and meal dose factor 432. The TDI 430 may be a TDI value that is modified in response to the pharmaceutical mode being set. Similarly, the meal dose factor 432 may be calculated as shown in Equation 2 above using a modified TDI, such as TDI 430, as well as a modified ICR. The modified ICR may be an Insulin-to-carbohydrate ratio that is modified in response to the pharmaceutical mode being set.

The bolus dosage may be an amount of medicament that is operable to compensate for changes to a user's blood glucose level caused by the ingested carbohydrates.

In the context of meal announcement 418, the bolus insulin delivered may be given by:

$$Safe\ Required\ IOB = \left( \frac{Current\ BG - (Setpoint + Elevation)}{Correction\ Factor} \right) \text{Equation 3,}$$

where current BG is a recent glucose measurement value, the setpoint is the target blood glucose setpoint for the user, the value of *Elevation* may be clinically determined and set to approximately 30 mg/dL or the like, and the *Correction Factor* is given by the 1800 rule as 1800/TDI, where TDI is a modified TDI. Note that the *Safe Required IOB* is similar to the *Correction* value calculated in Equation 1 except for the addition of the *Elevation* factor.

A bolus dosage to compensate for ingestion of the meal may be calculated as:

$$meal\ dose = x\%TDI + safe\ Required\ IOB - Current\ Total\ IOB \text{ Equation 4,}$$

where, the value of x may be, for example, 3 to 8, the safe required IOB is obtained from Equation 3, and the current total IOB is the determined amount of insulin-on-board for the user. For example, when the pharmaceutical mode is set, the processor may increase the value of x by 10% in the calculation of meal dose of Equation 4. A benefit of using Equations 3 and 4 in response to a meal announcement 418 is that no details of the meal composition, such as number of grams of carbohydrates, meal size, or the like, has to be provided. The factor x is chosen so as to not further reduce the user's glucose level when a small meal is ingested and to make a difference to the user's blood glucose when a large meal is ingested.

A secondary benefit of modifying the TDI is that basal delivery may also be adjusted because subsequent basal delivery is calculated based on the modified TDI. For example, by increasing basal delivery to further compensate for a meal at a more controllable pace. Since an effect of the pharmaceutical on the user is the under-delivery of medicament (or an over-delivery in case of decreased insulin resistance), the basal dosage may also be adjusted to also compensate for the under-delivery of medicament. The bolus dosage and the basal dosage are used in combination to provide the user with the appropriate amount of medicament needed by the user. Another potential modification may be to the bolus-basal split (or bolus split) of the total delivery of medicament to a user. A common split percentage is 50:50; however, this split percentage may be modified to increase the percentage of total medicament that is delivered to the user, such as 40% bolus and 60% basal, or some other percentages.

Using the modified TDI, the MDA may be operable to increase the amount of medicament delivered via basal delivery by relaxing medicament delivery constraints 436. The medicament delivery constraints 436 are, for example, MDA system constraints that may be used in limiting bolus delivery, basal delivery, or both, after the bolus delivery to further increase the system's ability to deliver insulin. Examples of MDA system constraints that may be relaxed include the one time 440, the IOB required 438, and/or the integral 442. "Relaxing a constraint" refers to altering the parameters governing the delivery of the medicament to allow for greater flexibility in aspects such as dosage. "Relaxing a constraint" may refer to increasing the range of allowable delivery actions by the MDA system. For example, relaxing a maximum bolus amount, the MDA system may deliver a higher maximum bolus amount.

The one time 440 constraint may limit the MDA from delivering greater than a multiple of the daily amount of medicament delivered by basal delivery to the user. For example, the one time 440 may be set by multiplying a user's current basal rate by the factory, where p may have a value between 6 - 9 when in pharmaceutical mode. Typically, when not in pharmaceutical mode the factor p is less than 6 and used to enable the MDA to compensate for a meal. Another constraint that may be modified is the required IOB that is typically may increased by about 40% when used to compensate for a meal, but when in pharmaceutical mode, the MDA may increase required IOB over the 40%, by for example, an additional 10-20%. A further constraint that may be changed is the integral 442, which is the amount of medicament (in particular insulin or GLP-1) delivered over a period of hours. More specifically, the integral 442 is the amount of insulin delivered over a number of basal hours; e.g., the amount of basal over a period of $m$ hours (such as 3), limited by $k$ (such as 3) times the period of hours $m$. A purpose of adjusting the integral 442 constraint is to limit the amount of insulin delivered over the $m$ hours by increasing the $k$ value to account for pharmaceutical mode, which relaxes (i.e., increases) this constraint. The integral 442 constraint is calculated by multiplying the variable $m$, which represents the number of basal hours (where the variable $m$ may be 36) by the variable $k$, and the variable $k$ may be 5-7. The medicament delivery constraints 436 may be referenced in terms of basal insulin needs.

An adaptive strategy to increase insulin dose by examining the blood glucose excursion post meals will be used as described with reference to the evaluation of the BG metrics at 406 and 412.

In an embodiment, the MDA may cause the calculated bolus dosage to be delivered by a wearable medicament delivery device. In another embodiment, the MDA may cause presentation of the calculated bolus dosage on a user interface for user confirmation, manual delivery of the bolus dosage by the user, or both. The bolus may be delivered at action 402. The time between receipt of the meal announcement 418 and the bolus delivery at 402 may be within seconds or minutes of one another.

After delivery at the bolus at 402, the MDA may begin monitoring BG metrics 404. For example, the MDA may note the user's glucose measurement value at time of receipt of the meal announcement 418 and set the value of the glucose measurement value as a reference glucose measurement value. The MDA may begin monitoring the user's blood glucose upon receipt of the first analyte measurement signal subsequent to the meal announcement 418 or subsequent to the bolus delivery at 402. For example, the first analyte measurement signal subsequent to the meal announcement 418 or subsequent to the bolus delivery at 402 may provide the glucose measurement value, information, or data useable to derive the glucose measurement value. In addition to evaluating metrics such as the number of times a glucose measurement value falls below or exceeds a first, second, or third glucose measurement threshold, other BG metrics may be included for evaluation at BG metrics 404 based on the number of past hypoglycemic events or hyperglycemic events.

A purpose of the process 400 is to provide additional insulin in response to a meal announcement 418 while in pharmaceutical mode because pharmaceuticals cause an under-delivery of insulin due to changes caused by the user's reaction to the pharmaceutical. The MDA may begin evaluating the user's BG metrics by determining whether the user's blood glucose metrics are higher than the second and third glucose measurement thresholds described above with reference to FIG. 3.

The MDA may still utilize the values of 180 mg/dL and 250 mg/dL as the respective second and third glucose measurement thresholds. In response to the BG metrics being high, and generating a "Yes, the BG metrics are high" response at decision block 406, the MDA may proceed to 408, 420, 422 and 424.

At 408, the MDA may set an increase by a set percentage, such as 10% or the like, for the TDI 430 and the meal dose factor 432.

At 420, the MDA may begin relaxing the onetime 440 medicament delivery constraints 436 by causing the factor $p$ to be increased in value by 1 for $Y$ number of hours. In this example, the factor $p$ increases the onetime 440 constraint thereby increasing an amount of medicament, such as insulin, to be delivered via basal delivery (i.e., $p$ x basal rate), and $Y$ is up to 3 hours or a typical post prandial period of time.

At 422, the MDA may begin relaxing the integral 442 constraint by increasing the factor $k$ by 1 for $Y$ hours, where Y is up to 3 hours or a typical post prandial period of time.

At 424, the MDA may begin relaxing the IOB required 438 constraint by increasing IOB required by $R$ %, where R represents a percentage between 0.0 and 60% with a typical increase being approximately 40 %.

Alternatively, if the response at 406 is "No, the BG metrics are not high," the process 400 proceeds to decision block 410 and 412. At 410, the MDA may decrease the TDI 430 and the meal dose factor 432 by a set percentage, such as 10% or the like, and at 412, the MDA determines whether the BG metrics are low, for example, by comparing the BG metrics to the first glucose measurement threshold (i.e., less than 70 mg/dL). In response to the BG metrics being low (i.e., a "Yes, the BG metrics are low" response being generated) at decision block 412, the MDA may proceed to 414, 416, and 426.

According to some examples, the MDA may relax settings of the one time 440 constraint by reducing the factor $p$ by 1 for $Y$ hours at 414, where $Y$ is up to 3 hours or a typical post prandial period of time. At 426, the MDA may relax settings for the integral 442 setting by reducing factor *k* by 1 for *Y* hours. In addition, the MDA may relax, at 416, the IOB required 438 setting by decreasing the IOB required by R %, the R may represent a percentage between 0 and 60%. The IOB required 438 may be compared to the *Safe Required IOB* calculated by Equation 3 to confirm the "safety" of the *Safe Required IOB.*

The above discussion of process 400 alluded to the process 400 was executed at every announcement of a meal, but that does not need to be the case. In some embodiments, the process 400 is run once daily, for example, during a user's largest meal of the day, or at a particular meal, such as breakfast, or for meals consumed during a particular time window, such as between 3 pm and 7 pm, or the like. Other alternatives may be for the process 400 to be executed every other day, weekly, or some other time period.

Although the example process 400 depicts a particular sequence of operations, the sequence may be altered without departing from the scope of the present disclosure. For example, some of the operations depicted may be performed in parallel or in a different sequence that does not materially affect the function of the routine. In other examples, different components of an example device or system that implements the process 400 may perform functions at substantially the same time or in a specific sequence.

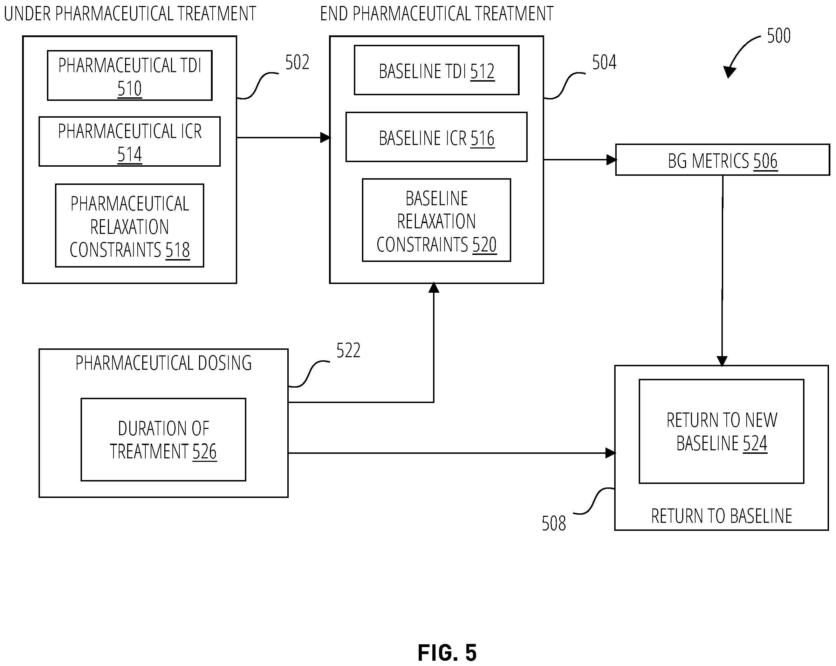

FIG. 5 illustrates an example process for ending pharmaceutical mode such as when a user completes a pharmaceutical treatment program in accordance with the disclosed subject matter.

When the user concludes a pharmaceutical treatment program, such as a corticosteroid therapy, and the AMD system ends operation in the pharmaceutical mode, the insulin requirements may be gradually brought to baseline needs (or a new baseline).

According to some examples, the MDA while in pharmaceutical mode may store the modified TDI, the modified ICR as well as the relaxed medicament delivery constraints, such as medicament delivery constraints 436, in a memory for later use. For example, the process 500 may store the values of pharmaceutical TDI 510, pharmaceutical ICR 514 and pharmaceutical relaxation constraints 518 in memory as values under pharmaceutical treatment 502.

A pharmaceutical treatment program may end, for example, when the duration settings 106a, 106b or 106C are met, when a user indicates that the pharmaceutical mode is terminated, or no longer needed or desired (e.g., an antibiotic regimen is no longer needed prior to meeting a set duration), or the like. When the pharmaceutical mode is terminated, the MDA may transition the pharmaceutical mode values of TDI, ICR and the medicament delivery constraints into values that correspond to values that represent settings prior to the user enter pharmaceutical mode. The prior settings may be those shown in end pharmaceutical treatment 504, such as baseline TDI 512, baseline ICR 516, and baseline relaxation constraints 520.

For example, the MDA may store in memory the TDI, ICR and the medicament delivery constraints from prior to the start of the pharmaceutical mode as baseline TDI 512, baseline ICR 516, and baseline relaxation constraints 520. The physiological changes caused by the MDA settings for pharmaceutical mode to MDA settings prior to pharmaceutical mode and the pharmaceutical treatment program are not going to be abrupt. However, the MDA settings do eventually have to change from the pharmaceutical mode settings to the settings that correspond to end pharmaceutical treatment 504. The settings that correspond to end pharmaceutical treatment 504 include baseline TDI 512, baseline ICR 516 and baseline relaxation constraints 520. When considering the eventual change from the pharmaceutical mode settings to the end pharmaceutical treatment 504 settings, the MDA may also take into account data, such as dosage amounts and the like from the pharmaceutical dosing 522

In order to facilitate the appropriate transition from the pharmaceutical mode back to the user's prior operating mode, the MDA may continue to monitor the BG metrics at 506. For example, the BG metrics evaluated at 506 may be similar to the metrics described with reference to the examples of FIG. 3 and FIG. 4. In addition, at 506, the MDA may compare the current analyte measurement signals to prior baseline settings, such as 512, baseline ICR 516 and baseline relaxation constraints 520. In addition, the duration of treatment 526 may also be provided to the MDA for consideration in adjusting or recalculating the end pharmaceutical treatment 504 settings from earlier baseline settings.

When determining the appropriate transition, the MDA may utilize a mathematical function, for example, such as slope interpolation or other function, for establishing a smooth transition from under pharmaceutical treatment 502 settings to end pharmaceutical treatment 504 settings that may extend over a few days, a week, or a number of weeks.

Eventually, the MDA is operable to enable a return to baseline 508 settings for TDI, ICR and the other MDA constraints. However, the MDA may note based on the evaluation of the BG metrics at 506 that the user has a new baseline for one or more of the MDA settings, and the MDA is operable to return to a new baseline 524. For example, the user's TDI may be higher than the user's baseline TDI 512, and as a result at 508, the MDA may set a new baseline TDI different from the user's previous TDI 512.

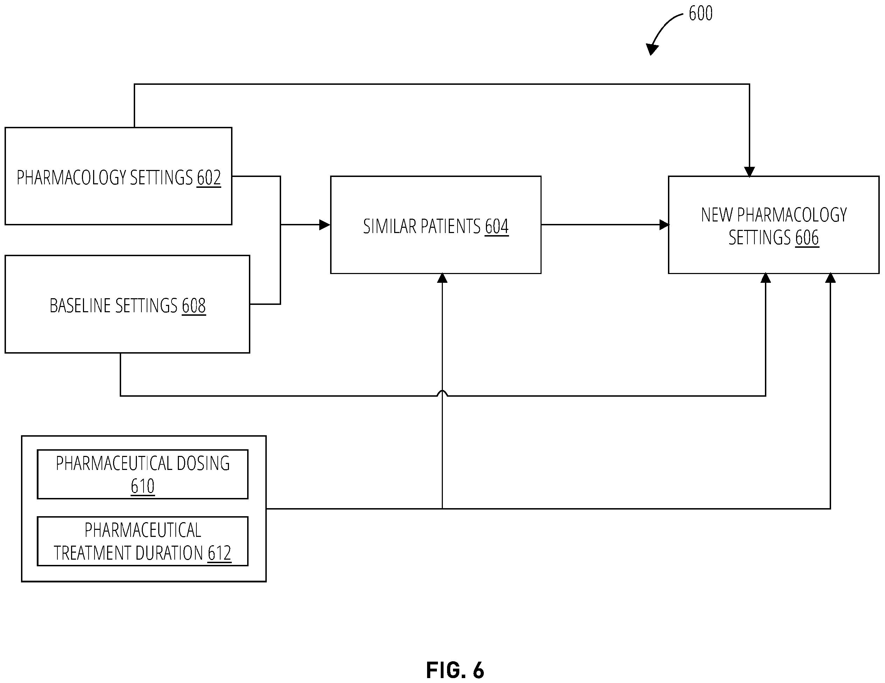

FIG. 6 illustrates a flowchart of an example of a learning process according to the disclosed subject matter.

It is anticipated that the system will utilize the information obtained from the analyte sensor and the response of the user's analytes to the actions of the pharmaceutical mode to learn what actions are optimal for the user. As such, the MDA is operable to catalog the elevated insulin requirements needed under a pharmaceutical treatment program for future use for the same patient. In addition, the data obtained by the MDA (such as the type of pharmaceutical, prescribed or recommended dosages, and the like) as well as the different MDA setting modifications may be anonymized and serve as a reference where a similar patient metric (e.g., persons of the same gender, similar weight, similar age, same diabetes type, duration of disease, TDI, weight, additional medications, exercise habits, meal habits, and the like) may be used to match the insulin requirements and MDA settings when the similar patient is under a pharmaceutical treatment program. The pharmacology setting learning process 600 may be executed as part of a cloud-based service.

For example, in pharmacology setting learning process 600, the pharmacology settings 602 and baseline settings 608 may be catalogued into a data set based on gender, weight, age, diabetes type and the like. The pharmacology setting learning process 600 may identify similar patients 604 in the data set and incorporate the current user's pharmacology settings 602 and baseline settings 608 into the data set with reference to the similar patients 604. In addition, the pharmaceutical dosing 610 (i.e., bolus dosages and basal dosages delivered) while in pharmaceutical mode and the pharmaceutical treatment duration 612 (i.e., the duration setting for the latest pharmaceutical mode) may also be cataloged with reference to similar patients 604 as well as being included in new pharmacology settings 606.

The new pharmacology settings 606 may be a determination of optimal MDA settings for a user that are based the BG metrics obtained in processes such as processes 300, 400 and 500. In addition to the BG metrics discussed with reference to those specific exemplary processes, the MDA and/or processors associated with cloud-based services (shown in a later example) may utilize other metrics, such as time in range or the like, for example, if there are a number of instances (e.g., 3 or more) of hypoglycemia, the MDA or cloud-based service may include a recommendation or setting for further reducing insulin deliveries during future pharmaceutical mode operations. The new pharmacology settings 606 may be stored for the particular user and may also be used generically for other users who start a pharmaceutical treatment program for a specific pharmaceutical. The MDA may query the new pharmacology settings 606 of the cloud-based service to obtain MDA setting recommendations for the user's pharmaceutical treatment program.

In summary, the baseline insulin needs and the elevated insulin needs are saved for future use. If the patient returns to a pharmaceutical treatment program, this information may be used as a reference. The elevated medicament needs identified from the pharmaceutical mode data may also be used for other similar patients. A catalog of these distinct aspects of a diabetes treatment plan or any other illness treatment plan requiring near continuous medicament delivery can quicken the establishment of new elevated doses.

While the aforementioned embodiments mostly related to a decrease in insulin sensitivity, it should be noted that they can also be adapted to account for an increase in insulin sensitivity. For example, some statins (e.g., atorvastatin, rosuvastatin), ACE inhibitors (e.g., Enalapril, Ramipril), or spironolactone may reduce insulin resistance, at least in some patients. Accordingly, the embodiments may be adapted to react to such insulin sensitivity increases, for example, by reducing the TDI and increasing the insulin to carbohydrate ratio (ICR).

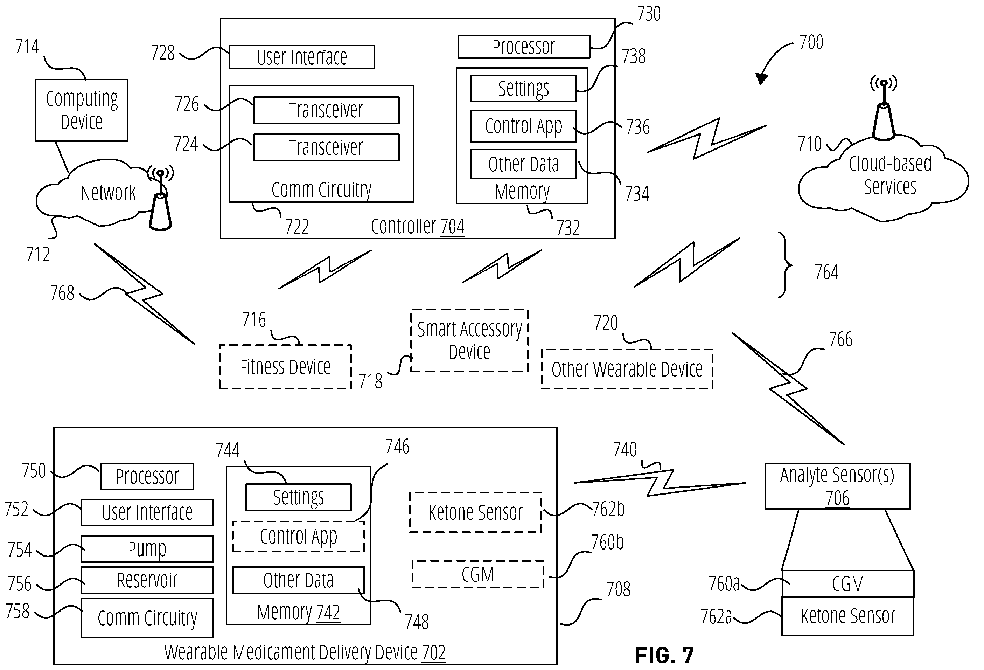

FIG. 7 illustrates an exemplary medicament system operable to implement the examples disclosed herein.

The medicament system 700 is suitable for delivering a liquid medicament, such as insulin to a user in accordance with the disclosed embodiments and discussion. While the system 700 is described with reference to delivery of insulin and the use of an MDA, the system 700 may be operable to implement a medicament regimen via the MDA using a number of different liquid or therapeutic drugs/medicaments. For example, a liquid medicament may be or include any medicament in liquid form capable of being administered by a medicament delivery device via a subcutaneous cannula, including, for example, insulin, glucagon-like peptide-1 (GLP-1), glucose-dependent insulinotropic polypeptide (GIP), pramlintide, glucagon, co-formulations of two or more of GLP-1, GIP, pramlintide, and insulin; as well as pain relief drugs, such as opioids or narcotics (e.g., morphine, or the like), methadone, blood pressure medicines, weight loss medicaments, chemotherapy drugs, fertility drugs, or the like. The medicament delivery system 700 may include at least a wearable medicament device 702, a controller 704, and an analyte sensor(s) 706.

In addition to the wearable medicament device 702, the controller 704, and the analyte sensor(s) 706, the medicament delivery system 700 may also include cloud-based services 710, a network 712, a computing device 714, a fitness device 716, a smart accessory device 718, and another wearable device 720.

The controller 704 may include housing(s) 708, a communication circuitry 722, a transceiver 724, a transceiver 726, a user interface 728, a processor 730, a memory 732, another data 734, a control application 736, and settings 738.

The wearable medicament delivery device 702 may include a memory 742, settings 744, a control application 746, another data 748, a processor 750, a user interface 752, a pump 754, a reservoir 756, communication circuitry 758, an optional continuous glucose monitor 760b, and an optional ketone sensor 762b. In a further example, the wearable medicament device 702 may optionally include a CGM 760b and a ketone sensor 762b, which may be removably incorporated or fully integrated within the wearable medicament device 702. For example, the continuous glucose monitor 760b and the ketone sensor 762b may be incorporated in the one or more housing(s) 708 of the wearable medicament delivery device 702.

The wearable medicament delivery device 702 may be a wearable device that is worn on the body of the user. The wearable medicament delivery device 702 may be directly coupled to a user (e.g., directly attached to exterior portion of skin of the user via an adhesive or the like). In an example, a surface of the housing(s) 708 of the wearable medicament delivery device 702 may include an adhesive to facilitate attachment to the skin of a user.

The wearable medicament delivery device 702 may include a processor 750. The processor 750 may be implemented in hardware, software, or any combination thereof. The processor 750 may, for example, be a microprocessor, a logic circuit, a field programmable gate array (FPGA), an application specific integrated circuit (ASIC) or a microprocessor coupled to a memory. The processor 750 may maintain a date and time as well as be operable to perform other functions (e.g., calculations or the like). The processor 750 may be operable to execute a control application 746 stored in the memory 742 that enables the processor 750 to direct operation of the wearable medicament delivery device 702. The control application 746 may, for example, be a medicament delivery application (MDA) that is operable to control insulin delivery to the user as describe herein. The memory 742 may hold settings 744 for a user, such as MDA settings, such as maximum insulin delivery, insulin sensitivity settings, insulin-to-carbohydrate ratio, total daily insulin (TDI) settings, medicament delivery constraints, such as integral, one time, and IOB required, and the like. The memory may also store other data 748, such as ketone values, total daily insulin (TDI) values, glucose measurement values from analyte sensor(s) 706 or controller 704, insulin dosage delivery amounts (both basal and bolus), and the like from previous minutes, hours, days, weeks, or months.

The analyte sensor(s) 706 may include a continuous glucose monitor 760a and/or a ketone sensor 762a. The analyte sensor(s) 706 may be operable to collect physiological condition data, such as ketone values, ketone values with a time stamp, glucose measurement values (also referred to herein as "blood glucose values," "glucose level," or "blood glucose"), glucose measurement values with a timestamp, trend information, and the like. In some examples, the ketone values and/or the glucose values are shared with the wearable medicament delivery device 702, the controller 704, or both. For example, the analyte sensor(s) 706 may provide the processor 730 and/or processor 750 with analyte measurement signals of measured or detected glucose levels of the user. The information or data provided by the analyte sensor(s) 706 may be used to modify a medicament delivery schedule and thereby cause the adjustment of MDA settings as described herein. In an additional example, the analyte sensor(s) 706 may include multiple sensors, such as a continuous glucose monitor 760a and ketone sensor 762a. Note that ketones may also be detected using a breath sensor (which is not shown but may be incorporated in the controller 704) or urine content sensor and stored in the respective memories 732 and 742 via a user input of the ketone levels; however, a subcutaneous ketone sensor(s) gives more accurate information and is more continuous. As described herein, the MDA and AMD system are described based on receiving ketone values subcutaneously. Use of a ketone breath sensor or urine sensor as part of or in addition to the analyte sensor(s) 706 may delay receipt of the ketone values and modifications to the system 700 may be made.

For example, the communication circuitry 758 of the wearable medicament delivery device 702 may be operable to communicate with the analyte sensor(s) 706 and the controller 704 as well as the devices 716, 718, and 720. The communication circuitry 758 may be operable to communicate via Bluetooth, cellular communication, near field communication (NFC) and/or other wireless protocols. The memory 742 may include random access memory (RAM), read only memory (ROM), optical storage, magnetic storage, removable storage media, solid state storage or the like.

The wearable medicament delivery device 702 may include a reservoir 756. The reservoir 756 may be operable to store liquid drugs, medicaments, or therapeutic agents suitable for automated delivery.

A fluid path to the user may be provided via tubing and a needle/cannula (not shown). The fluid path may, for example, include tubing coupling the wearable medicament wearable medicament delivery device 702 to the user (e.g., via tubing coupling a needle or cannula to the reservoir 756). The wearable medicament wearable medicament delivery device 702 may be operable based on control signals from the processor 750 to expel the liquid drugs, medicaments, or therapeutic agents, such as insulin, from the reservoir 756 to deliver doses of the drugs, medicaments, or therapeutic agents, such as the insulin, to the user via the fluid path. The processor 750 may be operable to cause insulin to be expelled from the reservoir 756 by the pump 754.

There may be one or more communications communication links 764 with one or more devices physically separated from the wearable medicament delivery device 702 including, for example, a controller 704 of the user and/or a caregiver of the user and/or an analyte sensor(s) 706. The communication links 764 may include any wired or wireless communication link operating according to any known communications protocol or standard, such as Bluetooth®, NFC, Wi-Fi, a near-field communication standard, a cellular standard, or any other wireless protocol. The analyte sensor(s) 706 may communicate with the wearable medicament delivery device 702 via a wireless communication link 740 and/or may communicate with the controller 704 via a wireless communication link 766.

The wearable medicament delivery device 702 may also include a user interface 752, such as an integrated display device for displaying information to the user, and in some embodiments, receiving information from the user. For example, the user interface 752 may include a touchscreen and/or one or more input devices, such as buttons, knob(s), or a keyboard that enable a user to provide an input.

In addition, the processor 750 may be operable to receive data or information from the analyte sensor(s) 706 as well as other devices that may be operable to communicate with the wearable medicament delivery device 702.

The wearable medicament delivery device 702 and/or the controller 704 may interface with a network 712 via communication link 768. The network 712 may include a local area network (LAN), a wide area network (WAN) or a combination therein. A computing device 714 may be interfaced with the network, and the computing device may communicate with the wearable medicament delivery device 702. The computing device 714 may be a healthcare provider device through which a user's controller 704 may interact to obtain information, store settings and the like.

The medicament system 700 may include an analyte sensor(s) 706 for sensing the levels of one or more analytes of a user. The analyte levels may be used as physiological condition data and be sent to the controller 704 and/or the wearable medicament delivery device 702. The sensor(s) 106 may be coupled to the user by, for example, adhesive or the like and may provide information or data on one or more medical conditions and/or physical attributes of the user.

The medicament system 700 may also include the controller 704. In the depicted example, the controller 704 may include a processor 730 and a memory 732. The controller 704 may be a special purpose device, such as a dedicated personal diabetes manager (PDM) device. The controller 704 may be a programmed general-purpose device that is a portable electronic device, such as any portable electronic device, smartphone, smartwatch, fitness device, tablet or the like including, for example, a dedicated processor, such as processor, a micro-processor, or the like. The controller 704 may be used to program or adjust operation of the wearable medicament delivery device 702 and/or the analyte sensor(s) 706. The processor 730 may execute processes to control the delivery of the medicament, drug, or therapeutic agent to the user for the purpose of managing a user's blood glucose and/or ketone levels. The processor 730 may also be operable to execute programming code stored in the memory 732. For example, the memory 732 may be operable to store a control application 736, such as an MDA for execution by the processor 730. The control application 736 may be responsible for controlling the wearable medicament device 702, including the automated delivery of medicament, a drug, or therapeutic agent based on recommendations and instructions from the MDA, such as those recommendations and instructions described herein.

The memory 742 may store one or more applications, such as control application 120, and settings 121 for the medicament device 702 like those described above. In addition, the memory 742 may be operable to store other data 748 and/or computer programs, such as medicament history, glucose measurement values over a period of time, total daily insulin values, ketone values, and the like. For example, the memory 742 is coupled to the processor 750 and operable to store programming instructions, such as the control application 746 and settings 744, and data, such as other data 748, related to a glucose level of a user and/or data related to an amount of insulin expelled by the wearable medicament wearable medicament delivery device 702.

The controller 704 may include a user interface (UI) 728 for communicating with the user. The user interface 728 may include a display, such as a touchscreen, for displaying information. The touchscreen may also be used to receive input when it is a touch screen. The user interface 728 may also include input elements, such as a keyboard, button, knob, or the like. In an operational example, the user interface 728 may include a touchscreen display (including a display and user input circuitry, such as touch sensitive circuits and the like) controllable by the processor 730 and be operable to present a graphical user interface.

The controller 704 may interface via a wireless communication link of the wireless communication links 764 with a network, such as a LAN or WAN or combination of such networks that provides one or more servers or cloud-based services 710 via communication circuitry 722. The communication circuitry 722, which may include transceivers 724 and 726, may be coupled to the processor 730. The communication circuitry 722 may be operable to transmit communication signals (e.g., command and control signals) to and receive communication signals (e.g., via transceivers 724 or 726) from the wearable medicament device 702 and the analyte sensor(s) 706. In an example, the communication circuitry 722 may include a first transceiver, such as transceiver 724, which may be a Bluetooth transceiver, and is operable to communicate with the communication circuitry 758 of the wearable medicament device 702, and a second transceiver, such as transceiver 726, that may be a cellular or Wi-Fi transceiver operable to communicate via the network 712 with computing device 714 or with cloud-based services 710.

The cloud-based services 710 may be operable to receive and store information obtained and utilized while an MDA was in pharmaceutical mode as well as baseline information based on user history information, such as glucose measurement values over a set period of time (e.g., days, months, years), a medicament history that includes insulin delivery amounts (both basal and bolus dosages) and insulin delivery, or the like.

Other devices, like smart accessory device 718 (e.g., a smartwatch or the like), fitness device 716 and other wearable device 720 may be part of the medicament system 700. These devices may communicate with the wearable medicament device 702 to receive information and/or issue commands to the wearable medicament device 702. These devices 716, 718, and 720 may execute computer programming instructions to perform some the control functions otherwise performed by processor 750 or processor 730. These devices 716, 718, and 720 may include user interfaces, such as touchscreen displays for displaying information such as current glucose level, medicament on board such as TDI, IOB, medicament delivery history (e.g., bolus doses and basal doses), or constraint settings, such as those described with reference to the examples of FIGs. 1A-6. The display may, for example, be operable to present a graphical user interface for providing input, such as setting pharmaceutical mode, duration settings, specific pharmaceutical information, and the like. These devices 716, 718, and 720 may also have wireless communication connections with the analyte sensor(s) 706 to directly receive glucose level data or receive in parallel a presentation of the graphical user interface as shown in FIG. 7.

The processor 730 is also operable to collect physiological condition data related to the user from sensors, such as the analyte sensor(s) 706 or heart rate data, for example, from the fitness device 716 or the smart accessory device 718.

In an example, the processor 730 executing the MDA may determine a dosage of medicament to be delivered based on the collected physiological condition of the user and/or a specific factor determined based on the subjective medicament need parameter. The processor 730 may output a control signal via one of the transceivers 726 or 724 to the wearable medicament device 702. The outputted signal may cause the processor 750 to deliver command signals to the pump 754 to deliver an amount of the determined dosage of medicament in the reservoir 756 to the user based on an output of the MDA. The processor 730 may also be operable to perform calculations to update or establish settings of the MDA as discussed as herein. Modifications to the MDA settings may be stored in the memory 732, for example, as settings 738.

A wearable medicament device 702, typically, has a lifecycle that is based on the amount of liquid drug that is stored in a reservoir 756 of the wearable medicament device 702 and/or the amount of the liquid drug delivered to the user. The MDA may use a number of parameters, such as glucose measurement values, total daily medicament, medicament onboard, and the like, when making the determination of an amount of the liquid drug to have delivered. In an operational example, the processor 730 of the controller 704 may be operable to receive evaluate the effectiveness of the MDA's control of the wearable medicament delivery device 702. For example, the processor 730 may be operable to utilize historical data accessible by the processor in an evaluation of past performance of the MDA and generate recommendations for adjusting settings of the MDA accordingly as described in more detail with reference to the examples in the figures.

Although the example processes 200-500 depict particular sequences of operations, the sequence may be altered without departing from the scope of the present disclosure. For example, some of the operations depicted may be performed in parallel or in a different sequence that does not materially affect the function of the routine. In other examples, different components of an example device or system that implements the routine may perform functions at substantially the same time or in a specific sequence.

Software related implementations of the techniques described herein, such as the processes examples described with reference to FIGs. 1-7 may include, but are not limited to, firmware, application specific software, or any other type of computer readable instructions that may be executed by one or more processors. The computer readable instructions may be provided via non-transitory computer-readable media. The various elements of the processes described with reference to the figures may be implemented in devices, apparatuses or systems that may include various hardware elements, software elements, or a combination of both. Examples of hardware elements may include structural members, logic devices, components, processors, microprocessors, circuits, processors, circuit elements (e.g., transistors, resistors, capacitors, inductors, and so forth), integrated circuits, application specific integrated circuits (ASIC), programmable logic devices (PLD), digital signal processors (DSP), field programmable gate array (FPGA), memory units, logic gates, registers, semiconductor device, chips, microchips, chip sets, and so forth. Examples of software elements may include software components, programs, applications, computer programs, application programs, system programs, software development programs, machine programs, operating system software, middleware, firmware, software modules, routines, subroutines, functions, processes, procedures, software interfaces, application program interfaces (API), instruction sets, computing code, computer code, code segments, computer code segments, words, values, symbols, or any combination thereof.

Some examples of the disclosed device or processes may be implemented, for example, using a storage medium, a computer-readable medium, or an article of manufacture which may store an instruction or a set of instructions that, if executed by a machine (i.e., processor or controller), may cause the machine to perform a method and/or operation in accordance with examples of the disclosure. Such a machine may include, for example, any suitable processing platform, computing platform, computing device, processing device, computing system, processing system, computer, processor, or the like, and may be implemented using any suitable combination of hardware and/or software. The computer-readable medium or article may include, for example, any suitable type of memory unit, memory, memory article, memory medium, storage device, storage article, storage medium and/or storage unit, for example, memory (including non-transitory memory), removable or non-removable media, erasable or non-erasable media, writeable or re-writeable media, digital or analog media, hard disk, floppy disk, Compact Disk Read Only Memory (CD-ROM), Compact Disk Recordable (CD-R), Compact Disk Rewriteable (CD-RW), optical disk, magnetic media, magneto-optical media, removable memory cards or disks, various types of Digital Versatile Disk (DVD), a tape, a cassette, or the like. The instructions may include any suitable type of code, such as source code, compiled code, interpreted code, executable code, static code, dynamic code, encrypted code, programming code, and the like, implemented using any suitable high-level, low-level, object-oriented, visual, compiled and/or interpreted programming language. The non-transitory computer readable medium embodied programming code may cause a processor when executing the programming code to perform functions, such as those described herein.

Certain examples of the present disclosure were described above. It is, however, expressly noted that the present disclosure is not limited to those examples, but the intention is that additions and modifications to what was expressly described herein are also included within the scope of the disclosed examples. Moreover, it is to be understood that the features of the various examples described herein were not mutually exclusive and may exist in various combinations and permutations, even if such combinations or permutations were not made express herein, without departing from the spirit and scope of the disclosed examples. In fact, variations, modifications, and other implementations of what was described herein will occur to those of ordinary skill in the art without departing from the spirit and the scope of the disclosed examples. As such, the disclosed examples are not to be defined only by the preceding illustrative description.

Program aspects of the technology may be thought of as "products" or "articles of manufacture" typically in the form of executable code and/or associated data that is carried on or embodied in a type of non-transitory, machine readable medium. The present disclosure furthermore relates to computer programs comprising instructions (also referred to as computer programming instructions) to perform the aforementioned functionalities. The instructions may be executed by a processor. The instructions may also be performed by a plurality of processors for example in a distributed computer system. The computer programs of the present disclosure may be for example preinstalled on, or downloaded to the medicament delivery device, management device, fluid delivery device, e.g. their storage. Although the aforementioned description distinguishes between a system and a device, that a device according to the claims may also be a system.

Storage type media include any or all of the tangible memory of the computers, processors or the like, or associated modules thereof, such as various semiconductor memories, tape drives, disk drives and the like, which may provide non-transitory storage at any time for the software programming. It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, novel subject matter lies in less than all features of a single disclosed example. Thus, the following claims are hereby incorporated into the Detailed Description, with each claim standing on its own as a separate example. In the appended claims, the terms "including" and "in which" are used as the plain-English equivalents of the respective terms "comprising" and "wherein," respectively. Moreover, the terms "first," "second," "third," and so forth, are used merely as labels and are not intended to impose numerical requirements on their objects.

The foregoing description of examples has been presented for the purposes of illustration and description. It is not intended to be exhaustive or to limit the present disclosure to the precise forms disclosed. Many modifications and variations are possible considering this disclosure. It is intended that the scope of the present disclosure be limited not by this detailed description, but by the claims appended hereto. Future filed applications claiming priority to this application may claim the disclosed subject matter in a different manner and may generally include any set of one or more features as variously disclosed or otherwise demonstrated herein.

Although the present invention is defined in the attached claims, it should be understood that the present invention can also (alternatively) be defined in accordance with the following embodiments:

1. A medicament delivery system, comprising:

a processor; and
a memory storing instructions including a medicament delivery application that, when executed by the processor, configure the processor to:

receive an indication to enter pharmaceutical mode;
modify settings of the medicament delivery application in response to the received indication, wherein the modified settings are based on an anticipated increase in medicament amounts due to increased medicament resistance; and
determine amounts of medicament to deliver to a user based on the modified settings.

2. The medicament delivery system of embodiment 1, wherein the instructions further cause the processor to:

receive a plurality of analyte levels of the user over a period of time;
evaluate the received plurality of analyte levels with respect to a plurality of analyte metrics; and
based on results of the evaluation, adjust total daily medicament settings and medicament to carbohydrate ratio.

3. The medicament delivery system of embodiment 2, wherein the processor, when evaluating the received plurality of analyte levels with respect to the plurality of analyte metrics, is operable to:
determine one or more of:

a number of the received plurality of analyte values that are below a first threshold,
a number of the received plurality of analyte values that are over a second threshold,
a number of the received plurality of analyte values that are over a third threshold,

a mean value of the received plurality of analyte in a first window of time, or
a mean value of the received plurality of analyte in a second window of time.

4. The medicament delivery system of embodiment 1, further comprising:
a user interface, the user interface configured to:
present a plurality of specific time durations.
5. The medicament delivery system of embodiment 4, wherein the processor is further operable to:
receive a time duration in response a selection of the time duration from the plurality of specific time durations.
6. The medicament delivery system of embodiment 1, further comprising:
a user interface, the user interface configured to:
present a start date prompt and an end date prompt.
7. The medicament delivery system of embodiment 6, wherein the processor is further operable to:

receive a start date entered into the start date prompt;
receive an end date entered into the end date prompt; and
determine a time duration based on the entered start date and the entered end date.

8. The medicament delivery system of embodiment 1, wherein the processor, when modifying settings of the medicament delivery application in response to the received indication, is further operable to:

evaluate current settings of the medicament delivery application with respect to a time duration included in the received indication,
evaluate current settings of the medicament delivery application reference to a listing of pharmaceuticals and their effects on blood glucose levels, and
determine which of the current settings of the medicament delivery application is to be modified.

9. The medicament delivery system of embodiment 1, wherein the processor is further operable to:
receive specific pharmaceutical information, wherein the specific pharmaceutical information includes at least one of:
a name (exact name or generic name) of the pharmaceutical, a dosage of the pharmaceutical, a frequency of dosages of the pharmaceutical, or quantity of pharmaceuticals.
10. The medicament delivery system of embodiment 1, wherein the modified settings include at least one or more of total daily insulin, insulin-to-carbohydrate ratio, or a medicament delivery constraint.
11. The medicament delivery system of embodiment 1, further comprising:
a wearable medicament delivery device including:

a reservoir storing a medicament; and
a pump coupled to the reservoir and operable to expel a determined amount of medicament from the reservoir based on the modified settings.

12. The medicament delivery system of embodiment 1, further comprising:
an analyte sensor, wherein the analyte sensor is operable to deliver an analyte measurement signal to the processor.
13. The medicament delivery system of embodiment 1, wherein the increased medicament resistance is caused due the presence of a pharmaceutical in blood of a user.
14. A controller of a medicament delivery system, comprising:

a processor; and
a memory storing instructions including a medicament delivery application that, when executed by the processor, configure the processor to:

receive a notification, while in a pharmaceutical mode of operation for the medicament delivery application;
in response to the notification, calculate a bolus dosage using a first medicament delivery application setting and a second medicament delivery application setting;
monitor analyte measurement signals with reference to one or more blood glucose metrics;
based on the monitoring, modify the first medicament delivery application setting and modify the second medicament delivery application setting according to pharmaceutical mode settings, wherein the modified first medicament delivery application setting and the modified second medicament delivery application setting have an inverse relationship.

15. The controller of embodiment 14, wherein the first medicament delivery application setting is total daily insulin and the second medicament delivery application setting is insulin-to-carbohydrate ratio.

16. The controller of embodiment 15, wherein the received notification is a bolus request.

17. The controller of embodiment 14, wherein the processor when executing the stored instructions including the medicament delivery application, is further configured, when monitoring the analyte measurement signals with reference to the one or more blood glucose metrics, to:

determine whether the analyte measurement signals are low; and

in response to the low determination, the modifying of the first medicament delivery application setting is reducing the first medicament delivery application setting by a set percentage and the modifying of the second medicament delivery application setting is increasing the second medicament delivery application setting by another set percentage.

18. The controller of embodiment 14, wherein the processor when executing the stored instructions including the medicament delivery application, is further configured, when monitoring the analyte measurement signals with reference to the one or more blood glucose metrics, to:

determine whether the analyte measurement signals are high; and

in response to the high determination, the modifying of the first medicament delivery application setting is increasing the first medicament delivery application setting by a set percentage and the modifying of the second medicament delivery application setting is reducing the second medicament delivery application setting by another set percentage.

19. The controller of embodiment 14, wherein, when the received notification is a meal announcement, the processor when executing the stored instructions including the medicament delivery application, is further configured to: modify a medicament delivery constraint.

20. A controller for a wearable medicament delivery device, comprising:

a processor; and

a memory storing a medicament delivery application and medicament delivery application settings, wherein the medicament delivery application is operable to cause the processor to:

receive an indication that a pharmaceutical mode of the medicament delivery application is terminated, wherein medicament delivery application settings are set to pharmaceutical mode settings that control medicament delivery while in pharmaceutical mode; and

transition the medicament delivery application settings from the pharmaceutical mode settings to adjusted medicament delivery application settings.

**Claims**

1. A medicament delivery system, comprising:

a processor; and

a memory storing instructions including a medicament delivery application that, when executed by the processor, configure the processor to:

receive an indication to enter pharmaceutical mode;

modify settings of the medicament delivery application in response to the received indication, wherein the modified settings are based on an anticipated change in medicament amounts required by a user due to a change in medicament resistance; and determine amounts of medicament to deliver to a user based on the modified settings.

2. The medicament delivery system of claim 1, wherein the instructions further cause the processor to:

receive a plurality of analyte levels of the user over a period of time;

evaluate the received plurality of analyte levels with respect to a plurality of analyte metrics; and

based on results of the evaluation, adjust total daily medicament settings and

medicament to carbohydrate ratio.

3. The medicament delivery system of claim 2, wherein the processor, when evaluating the received plurality of analyte levels with respect to the plurality of analyte metrics, is operable to:
determine one or more of:

a number of the received plurality of analyte values that are below a first threshold,
a number of the received plurality of analyte values that are over a second threshold,
a number of the received plurality of analyte values that are over a third threshold,
a mean value of the received plurality of analyte in a first window of time, or
a mean value of the received plurality of analyte in a second window of time.

4. The medicament delivery system of any one of claims 1 to 3, further comprising:
a user interface, the user interface configured to:
present a plurality of specific time durations, more specifically wherein the processor is further operable to:
receive a time duration in response a selection of the time duration from the plurality of specific time durations.

5. The medicament delivery system of any one of claims 1 to 4, further comprising:
a user interface, the user interface configured to:
present a start date prompt and an end date prompt, more specifically wherein the processor is further operable to:

receive a start date entered into the start date prompt;
receive an end date entered into the end date prompt; and
determine a time duration based on the entered start date and the entered end date.

6. The medicament delivery system of any one of claims 1 to 5, wherein the processor, when modifying settings of the medicament delivery application in response to the received indication, is further operable to:

evaluate current settings of the medicament delivery application with respect to a time duration included in the received indication,
evaluate current settings of the medicament delivery application reference to a listing of pharmaceuticals and their effects on blood glucose levels, and
determine which of the current settings of the medicament delivery application is to be modified.

7. The medicament delivery system of any one of claims 1 to 6, wherein the processor is further operable to:
receive specific pharmaceutical information, wherein the specific pharmaceutical information includes at least one of:
a name of the pharmaceutical, a dosage of the pharmaceutical, a frequency of dosages of the pharmaceutical, or quantity of pharmaceuticals.

8. The medicament delivery system of any one of claims 1 to 7, wherein the modified settings include at least one or more of total daily insulin, insulin-to-carbohydrate ratio, or a medicament delivery constraint.

9. The medicament delivery system of any one of claims 1 to 8, further comprising:
a wearable medicament delivery device including:

a reservoir storing a medicament; and
a pump coupled to the reservoir and operable to expel a determined amount of medicament from the reservoir based on the modified settings.

10. The medicament delivery system of any one of claims 1 to 9, further comprising:
an analyte sensor, wherein the analyte sensor is operable to deliver an analyte measurement signal to the processor, which the processor processes to analyte values; or wherein the analyte sensor is operable to deliver an analyte values to the processor.

11. The medicament delivery system of any one of claims 1 to 10, wherein the increased medicament resistance is caused due the presence of a pharmaceutical in blood of a user.

12. A controller of a medicament delivery system, comprising:

a processor; and

a memory storing instructions including a medicament delivery application that, when executed by the processor, configure the processor to:

receive a notification, while in a pharmaceutical mode of operation for the medicament delivery application;

in response to the notification, calculate a bolus dosage using a first medicament delivery application setting and a second medicament delivery application setting;

monitor analyte measurement signals with reference to one or more blood glucose metrics;

based on the monitoring, modify the first medicament delivery application setting and modify the second medicament delivery application setting according to pharmaceutical mode settings, wherein the modified first medicament delivery application setting and the modified second medicament delivery application setting have an inverse relationship.

13. The controller of claim 12, wherein the first medicament delivery application setting is total daily insulin, the second medicament delivery application setting is insulin-to-carbohydrate ratio and/or the received notification is a bolus request.

14. The controller of any one of claims 12 or 13, wherein the processor when executing the stored instructions including the medicament delivery application, is further configured, when monitoring the analyte measurement signals with reference to the one or more blood glucose metrics, to:

determine whether the analyte measurement signals are low; and

in response to the low determination, the modifying of the first medicament delivery application setting is reducing the first medicament delivery application setting by a set percentage and the modifying of the second medicament delivery application setting is increasing the second medicament delivery application setting by another set percentage; or

in response to the high determination, the modifying of the first medicament delivery application setting is increasing the first medicament delivery application setting by a set percentage and the modifying of the second medicament delivery application setting is reducing the second medicament delivery application setting by another set percentage.

15. The controller of any one of claims 12 to 14, wherein the processor is configured to:

receive an indication that a pharmaceutical mode of the medicament delivery application is terminated, wherein medicament delivery application settings are set to pharmaceutical mode settings that control medicament delivery while in pharmaceutical mode; and

transition the medicament delivery application settings from the pharmaceutical mode settings to adjusted medicament delivery application settings.

108a

100a

102

SET PHARMACEUTICAL MODE

DURATION

104

110

SPECIFIC PHARMACEUTICAL
INFORMATION

| 1 WEEK | 2 WEEKS |
| 3 WEEKS | 4 WEEKS |

106a

**FIG. 1A**

100b

108b

102 — SET PHARMACEUTICAL MODE

104 — DURATION

110 — SPECIFIC PHARMACEUTICAL INFORMATION

| 2 DAYS | 3 DAYS |
| 4 DAYS | 5 DAYS |
| 6 DAYS | 7 DAYS |

106b

**FIG. 1B**

100C

108C

102

SET PHARMACEUTICAL MODE

DURATION

104

110

SPECIFIC PHARMACEUTICAL
INFORMATION

106C

START DATE

END DATE

**FIG. 1C**

200

RECEIVE AN INDICATION TO ENTER PHARMACEUTICAL MODE 202

MODIFY SETTINGS OF A MEDICAMENT DELIVERY APPLICATION IN RESPONSE TO THE RECEIVED INDICATION 204

DETERMINE AMOUNTS OF MEDICAMENT TO DELIVER TO A USER BASED ON THE MODIFIED SETTINGS 206

**FIG. 2**

**FIG. 3**

EP 4 542 560 A1

400

INCREASE BY SET PERCENTAGE — 408

DECREASE BY SET PERCENTAGE — 410

BG METRICS HIGH? 406 — YES / NO

BG METRICS 404

DELIVER BOLUS 402

BOLUS CALCULATOR 434

TDI 430

MEAL DOSE FACTOR 432

MEAL ANNOUNCEMENT 418

INCREASE P BY 1 FOR Y HOURS FOR RELAXED ONE TIME SETTING — 420

INCREASE K BY 1 FOR Y HOURS FOR RELAXED INTEGRAL SETTING — 422

INCREASE IOB REQUIRED BY R % FOR RELAXED SETTING — 424

REDUCE P BY 1 FOR Y HOURS FOR RELAXED ONE TIME SETTING — 414

REDUCE K BY 1 FOR Y HOURS FOR RELAXED INTEGRAL SETTING — 426

DECREASE IOB REQUIRED BY R % FOR RELAXED SETTINGS — 416

BG METRICS LOW? 412 — YES / NO

RETURN TO 442 — 428

MEDICAMENT DELIVERY CONSTRAINTS 436

IOB REQUIRED 438

ONE TIME 440

INTEGRAL 442

FIG. 4

UNDER PHARMACEUTICAL TREATMENT

END PHARMACEUTICAL TREATMENT

500

PHARMACEUTICAL TDI 510

502

BASELINE TDI 512

504

PHARMACEUTICAL ICR 514

BASELINE ICR 516

BG METRICS 506

PHARMACEUTICAL RELAXATION CONSTRAINTS 518

BASELINE RELAXATION CONSTRAINTS 520

PHARMACEUTICAL DOSING

522

DURATION OF TREATMENT 526

RETURN TO NEW BASELINE 524

508

RETURN TO BASELINE

**FIG. 5**

FIG. 6

FIG. 7

Europäisches
Patentamt

European
Patent Office

Office européen
des brevets

# EUROPEAN SEARCH REPORT

**Application Number**

EP 24 20 7459

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/287985 A1 (ESTES MARK C [US] ET AL) 13 December 2007 (2007-12-13) <br> * The whole document, in particular: Paragraphs [0008], [0012], [0027], [0028], [0031], [0032], [0037], [0040], [0043], [0045], [0050], [0053] - [0055], [0057], [0060] - [0063], [0066] - [0068], [0070], [0080], [0082]; Figures 1, 3A - D, 4 - 6. * | 1-15 | INV. <br> G16H20/17 <br> A61B5/145 <br> A61M5/172 <br> G16H40/63 |
| A | US 2010/198021 A1 (ALFERNESS CLIFTON A [US] ET AL) 5 August 2010 (2010-08-05) <br> * The whole document, in particular: Paragraphs [0013], [0055], [0071], [0074], [0113] - [0119], [0122], [0123]; Figures 6 - 9, 19 - 21, 28. * | 1-15 | |
| A | CHAN J C N ET AL: "DRUG-INDUCED DISORDERS OF GLUCOSE METABOLISM MECHANISME AND MANAGEMENT", DRUG SAFETY, ADIS PRESS, AUCKLAND, NZ, vol. 15, no. 2, 1 August 1996 (1996-08-01) , pages 135-157, XP001041613, ISSN: 0114-5916, DOI: 10.2165/00002018-199615020-00005 <br> * The whole document. * | 1-15 | **TECHNICAL FIELDS SEARCHED (IPC)** <br><br> G16H <br> A61B <br> A61M |

The present search report has been drawn up for all claims

| Place of search | Date of completion of the search | Examiner |
|---|---|---|
| Munich | 26 February 2025 | Ruschke, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

....................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 7459

This annex lists the patent family members relating to the patent documents cited in the above-mentioned European search report.
The members are as contained in the European Patent Office EDP file on
The European Patent Office is in no way liable for these particulars which are merely given for the purpose of information.

26-02-2025

| Patent document cited in search report | Publication date | Patent family member(s) | | Publication date |
|---|---|---|---|---|
| US 2007287985 A1 | 13-12-2007 | AT | E344070 T1 | 15-11-2006 |
| | | AU | 2002366794 A1 | 09-07-2003 |
| | | CA | 2471007 A1 | 03-07-2003 |
| | | CA | 2736633 A1 | 03-07-2003 |
| | | DE | 60215856 T2 | 06-06-2007 |
| | | DK | 1458435 T3 | 19-03-2007 |
| | | EP | 1458435 A2 | 22-09-2004 |
| | | EP | 1759726 A2 | 07-03-2007 |
| | | JP | 2005512689 A | 12-05-2005 |
| | | US | 2003114836 A1 | 19-06-2003 |
| | | US | 2005245904 A1 | 03-11-2005 |
| | | US | 2007287985 A1 | 13-12-2007 |
| | | US | 2011028901 A1 | 03-02-2011 |
| | | US | 2011295341 A1 | 01-12-2011 |
| | | US | 2012035547 A1 | 09-02-2012 |
| | | US | 2012041414 A1 | 16-02-2012 |
| | | US | 2012041415 A1 | 16-02-2012 |
| | | WO | 03053498 A2 | 03-07-2003 |
| US 2010198021 A1 | 05-08-2010 | EP | 2378450 A2 | 19-10-2011 |
| | | US | 2010198021 A1 | 05-08-2010 |

EPO FORM P0459

For more details about this annex : see Official Journal of the European Patent Office, No. 12/82